(19) Europäisches Patentamt
European Patent Office
Office européen des brevets

(11) Veröffentlichungsnummer: **0 031 883**
**B1**

(12) # EUROPÄISCHE PATENTSCHRIFT

(45) Veröffentlichungstag der Patentschrift: **02.03.83**

(21) Anmeldenummer: **80106908.9**

(22) Anmeldetag: **08.11.80**

(51) Int. Cl.³: **A 61 K 31/415** // C07D303/04, C07D303/08, C07D301/00, C07D233/60

(54) **Antimikrobielle Mittel enthaltend Hydroxybutylimidazol-Derivate.**

(30) Priorität: **21.11.79 DE 2946957**

(43) Veröffentlichungstag der Anmeldung: **15.07.81 Patentblatt 81/28**

(45) Bekanntmachung des Hinweises auf die Patenterteilung: **02.03.83 Patentblatt 83/9**

(84) Benannte Vertragsstaaten: **BE CH DE FR GB IT LI NL SE**

(56) Entgegenhaltungen:
**EP-A-0 023 103**
**DE-A-1 940 388**
**DE-A-2 623 129**
**DE-A-2 736 122**
**CHEMICAL ABSTRACTS, Band 91, Nr. 7, 13-08-1979, Seite 709, Nr. 57005p. Columbus, Ohio, US**

(73) Patentinhaber: **BAYER AG, Zentralbereich Patente, Marken und Lizenzen, D-5090 Leverkusen 1, Bayerwerk (DE)**

(72) Erfinder: **Regel, Erik, Ing.-grad., Bergerheide 72a, D-5600 Wuppertal 1 (DE)**
Erfinder: **Büchel, Karl-Heinz, Prof. Dr., Dabringhausener Strasse 42, D-5093 Burscheid (DE)**
Erfinder: **Haller, Ingo, Dr., Viktoriastrasse 99, D-5600 Wuppertal 1 (DE)**
Erfinder: **Plempel, Manfred, Dr., Pahlkestrasse 5, D-5600 Wuppertal 1 (DE)**

EP 0 031 883 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Antimikrobielle Mittel, enthaltend Hydroxybutylimidazol-Derivate

Die vorliegende Erfindung betrifft neue Hydroxybutyl-imidazol-Derivate als Antimykotika.

Es ist bereits bekannt geworden, daß 1-(β-Aryl)-ethyl-imidazol-Derivate, wie insbesondere das 1-[2,4-Dichlor-β-(2,4-dichlorbenzyloxy)-phenethyl]-imidazolnatrium (MICONAZOL®), gute antimykotische Wirkung aufweisen (vergleiche DE-AS 1 940 388). Fungizide und/oder antimykotische Imidazolderivate sind auch aus der EP-A- 0 023 103 (nachveröffentlicht), C.A. 91 (1979), 5 7005 p, DE-A 2 736 122 und DE-A 2 623 129 bekannt geworden. Jedoch ist die Wirkung all dieser Verbindungen in vivo, wie insbesondere gegen Candida, nicht immer befriedigend.

Es wurde gefunden, daß die Hydroxybutyl-imidazol-Derivate der allgemeinen Formel

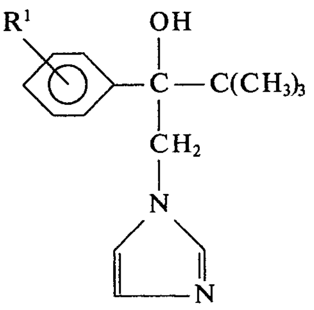

(I)

in welcher

$R^1$ für gegebenenfalls durch Wasserstoff, Halogen, Alkyl mit 1–2 Kohlenstoffatomen oder Alkoxy mit 1–2 Kohlenstoffatomen substituiertes Phenyl steht,

und/oder deren physiologisch verträglichen Säureadditions-Salze gute antimikrobielle, insbesondere antimykotische Eigenschaften aufweisen.

Überraschenderweise zeigen die als Wirkstoffe der erfindungsgemäßen Mittel verwendeten Hydroxybutyl-imidazol-Derivate neben einer guten antimykotischen in-vitro-Wirksamkeit eine bessere, therapeutisch nutzbare in-vivo-Wirksamkeit gegen Candida als das aus dem Stand der Technik bekannte 1-[2,4-Dichlor-β-(2,4-dichlorbenzyloxy)-phenethyl]-imidazolnatrium, welches ein anerkannt gutes Mittel gleicher Wirkungsrichtung ist. Die erfindungsgemäßen Wirkstoffe stellen somit eine wertvolle Bereicherung der Pharmazie dar.

Die als Wirkstoffe des erfindungsgemäßen Mittel zu verwendenen Hydroxybutyl-imidazol-Derivate sind durch die Formel (I) allgemein definiert.

Im einzelnen seien außer den bei den Herstellungsbeispielen genannten Verbindungen die folgenden Verbindungen der allgemeinen Formel (I) genannt:

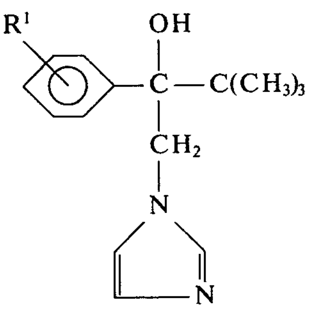

(I)

| $R^1$ |
|---|
| 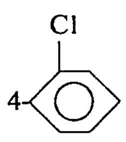 |
| 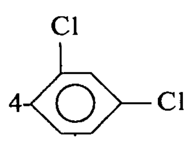 |

Die erfindungsgemäß zu verwendenden Wirkstoffe und deren Säureadditionssalze waren bisher noch nicht bekannt. Sie können jedoch gemäß einem eigenen hier nicht beanspruchten Vorschlag hergestellt werden, indem man Oxirane der Formel

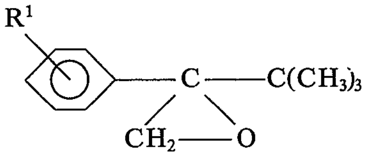

(II)

in welcher

$R^1$ die oben angegebene Bedeutung hat,

mit Imidazol in Gegenwart eines Alkalialkoholats, wie beispielsweise Natriummethylat, und in Gegenwart eines inerten organischen Lösungsmittels, wie beispielsweise Dimethylformamid, bei Temperaturen zwischen 30° C und 100° C umsetzt. Die Isolierung der Endprodukte erfolgt in allgemein üblicher Weise.

Die Oxirane der Formel (II) sind noch nicht bekannt. Sie werden erhalten, indem man entsprechende Ketone der Formel

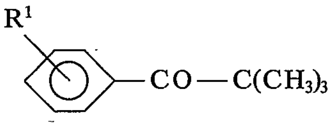

(III)

in welcher

$R^1$ die oben angegebene Bedeutung hat,

*α*) mit Dimethyloxosulfonium-methylid der Formel

$$(CH_3)_2\overset{\oplus}{S}O\overset{\ominus}{C}H_2 \qquad (IV)$$

in an sich bekannter Weise in Gegenwart eines Verdünnungsmittels, wie beispielsweise Dimethylsulfoxid, bei Temperaturen zwischen 20 und 80° C umsetzt (vergleiche hierzu auch die Angaben in JACS 87, 1363–1364 (1965)), oder

*β*) mit Trimethylsulfonium-methylsulfat der Formel

$$(CH_3)_3\overset{\oplus}{S}CH_3SO_4^{\ominus} \qquad (V)$$

in an sich bekannter Weise in Gegenwart eines Zweiphasensystems und gegebenenfalls in Gegenwart eines Phasentransferkatalysators bei Temperaturen zwischen 0 und 100° C umsetzt (vergleiche auch die angaben in Heterocycles 8, 397 (1977)).

Weder die Oxirane der Formel (II) noch ihr vorstehend genanntes Herstellungsverfahren werden hier beansprucht.

Die Ketone der Formel (III) sind allgemein bekannte Verbindungen der organischen Chemie.

Das Dimethyloxosulfonium-methylid der Formel (IV) ist ebenfalls bekannt und wird in situ eingesetzt, indem man Trimethyloxosulfoniumjodid mit Natriumhydrid (vergleiche auch die o. g. Literaturstelle) bzw. Natriumamid umsetzt.

Das Trimethylsulfonium-methylsulfat der Formel (V) ist auch bekannt und wird in situ eingesetzt, indem man Dimethylsulfid mit Dimethylsulfat umsetzt (vergleiche auch die o. g. Literaturstelle).

Die nach den Verfahren (*α*) und (*β*) erhältlichen Oxirane der Formel (II) können auch direkt, d. h. ohne Isolierung, weiter zu den Endprodukten der Formel (I) umgesetzt werden.

Die erfindungsgemäß zu verwendenden Verbindungen der Formel (I) können auch erhalten werden, wenn man z. B. entsprechende Imidazolylmethyl-phenyl-ketone in bekannter Art und Weise mit tert.-Butylmagnesiumbromid umsetzt oder wenn man die entsprechenden 1-Halogen-1-hydroxy-2-phenyl-3,3-dimethyl-butane in bekannter Art und Weise mit Imidazol umsetzt.

Zur Herstellung von Säureadditionssalzen der Verbindungen der Formel (I) kommen alle physiologisch verträglichen Säuren in Frage. Hierzu gehören vorzugsweise die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure. Salizylsäure, Sorbinsäure, Milchsäure sowie Sulfonsäuren, wie z. B. p-Toluolsulfonsäure und 1,5-Naphthalindisulfonsäure.

Die Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäß verwendbaren Verbindungen der Formel (I), ihre Säureadditions-Salze und Metallsalz-Komplexe weisen starke antimykotische Wirkungen auf. Sie besitzen ein sehr breites antimykotisches Wirkungsspektrum, insbesondere gegen Dermatophyten und Sproßpilze sowie bisphasische Pilze, zum Beispiel gegen Candida-Arten, wie Candida albicans, Epidermophyton-Arten, wie Epidermophyton floccosum, Aspergillus-Arten, wie Aspergillus niger und Aspergillus fumigatus, wie Trichophyton-Arten, wie Trichophyton mentagrophytes, Microsporon-Arten, wie Microsporon felineum sowie Penicillin-Arten, wie Penicillium commune. Die Aufzählung dieser Mikroorganismen ist eine beispielhafte Erläuterung der bekämpfbaren Keime.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiet in der Tiermedizin können beispielsweise aufgeführt werden: Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabellen, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder ½, ⅓ oder ¼ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glucose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quaternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyäthylenglykole oder Gemische der unter (a) bis (i) aufgeführten Stoffe.

Den Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z. B. Polyäthylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes oder Gele können neben dem oder den Wirkstoffen die üblichen Trägerstoffe enthalten, z. B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Äthylalkohol, Isopropylalkohol, Äthylcarbonat, Äthylacetat, Benzylalkohol, Benzylenzoat, Propylenglykol, 1,3-Butylenglykol, Dimethylformamid, Oele, insbesondere Baumwollsaatöl, Erdnußöl, Maiskeimöl, Olivenöl, Ricinusöl und

Sesaöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Äthylalkohol, Propylenglykol, Suspendiermittel, z. B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z. B. Pfefferminzöl und Eukalyptusöl und Süßmittel, z. B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten, soweit sie für die vorstehend genannten Einsatzzwecke bekannt sind.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die erfindungsgemäßen Wirkstoffe sowie pharmazeutischen Zubereitungen, die einen oder mehrere erfindungsgemäße Wirkstoffe enthalten, sind in der Human- und Veterinärmedizin zur Verhütung, Besserung und/oder Heilung der oben angeführten Erkrankungen geeignet.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Herstellungsbeispiele

### Beispiel 1

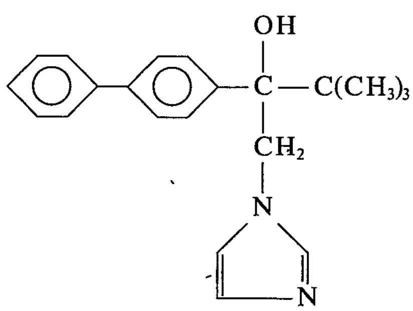

Eine Lösung von 22,2 g (0,088 Mol) 2-(4-Biphenylyl)-2-(tert.-butyl)-oxiran in 150 ml Dimethylformamid wird in eine Lösung von 6,4 g (0,117 Mol) Natriummethylat und 13,2 g (0,2 Mol) Imidazol in 40 ml Methylalkohol getropft. Nach 6stündigem Erwärmen auf 60° C wird das Gemisch im Vakuum eingeengt, auf 3000 ml Wasser gegeben, die ausgeschiedene Kristallmasse in Methylenchlorid gelöst und die Lösung mit Wasser gewaschen. Die Lösung wird über Natriumsulfat getrocknet und im Vakuum eingedampft. Die Kristalle werden mit Ether verrührt. Man erhält 20,5 g (73% der Theorie) 1-(4-Biphenylyl)-1-(tert.-butyl)-2-(imidazol-1-yl)-ethan-1-ol vom Schmelzpunkt 184° C.

Herstellung des Ausgangsproduktes

(neues Zwischenprodukt der Formel (II))

(II-1)

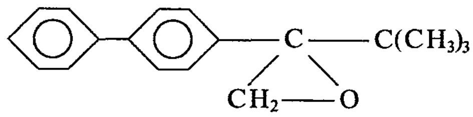

3,6 g (0,12 Mol) 80%iges Natriumhydrid und 26,4 g (0,12 Mol) Trimethyloxosulfoniumjodid werden unter Rühren tropfenweise mit 120 ml Dimethylsulfoxid versetzt. Anschließend wird eine Lösung von 23,8 g (0,1 Mol) 4-Biphenylyl-tert.-butyl-keton in 150 ml Dimethylsulfoxid unter Rühren zugetropft. Das Gemisch wird 3 Stunden auf 50° C erwärmt, danach werden unter Kühlen bei 20° C 500 ml Wasser zugetropft. Die ausgeschiedenen Kristalle werden in Chloroform gelöst, die Lösung wird mit Wasser gewaschen, die Chloroformlösung über Natriumsulfat getrocknet und am Rotationsverdampfer eingedampft. Die Kristallmasse wird mit Petrolether verrührt und abgesaugt. Man erhält 22,3 g (88% der Theorie) 2-(4-Biphenylyl)-2-(tert.-butyl)-oxiran vom Schmelzpunkt 78° C.

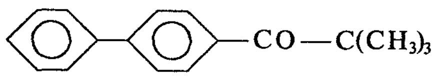

6 g (0,2 Mol) 80%iges Natriumhydrid werden in 100 ml Tetrahydrofuran vorgelegt. Unter Rühren werden 44,4 g (0,2 Mol) 4-Biphenylyl-isopropyl-keton eingetragen. Nach Abklingen der Gasentwicklung wird 3 Stunden auf 40° C erwärmt, dann werden 28,8 g (0,2 Mol) Methyljodid zugetropft. Nach 48 Stunden wird die Lösung filtriert und im Vakuum eingedampft; der Rückstand wird in Essigester gelöst, die Lösung mit Wasser gewaschen, über Natriumsulfat getrocknet und erneut im Vakuum eingedampft. Nach Verrühren der Kristallmasse mit Waschbenzin erhält man 40,5 g (85% der Theorie) 4-Biphenylyl-tert.-butyl-keton von Schmelzpunkt 98° C.

Beispiel 2

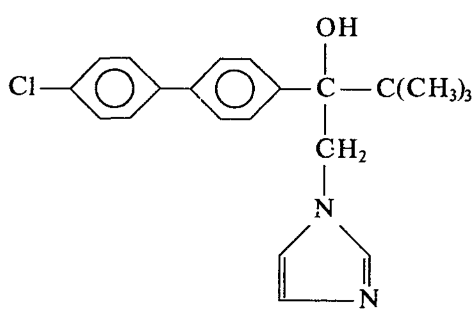

Entsprechend Beispiel 1 erhält man aus 5,6 g (0,104 Mol) Natriummethylat, 40 ml Methylalkohol, 12,2 g(0,18 Mol) Imidazol, 22,8 g (0,08 Mol) 2-(4-p-Chlorbiphenylyl)-2-(tert.-butyl)-oxiran und 150 ml Dimethylformamid 23,5 g (83% der Theorie) 1-(4-p-Chlorbiphenylyl)-1-(tert.-butyl)-2-(imidazol-1-yl)-ethan-1-ol vom Schmelzpunkt 164° C.

Herstellung des Ausgangsproduktes

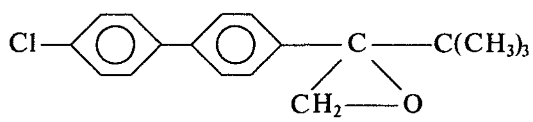

Entsprechend Beispiel 1 erhält man aus 3,6 g (0,12 Mol) Natriumhydrid (80%ig) 26,4 g (0,12 Mol) Trimethyloxosulfoniumjodid, 27,3 g (0,1 Mol) 4-p-Chlorbiphenylyl)-tert.-butyl-keton und 220 ml Dimethylsulfoxid nach Verrühren des Rohproduktes mit Diisopropylether 17,3 g (60% der Theorie) 2-(4-p-Chlorbiphenylyl)-2-(tert.-butyl)-oxiran vom Schmelzpunkt 118° C.

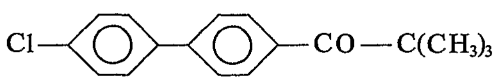

Entsprechend Beispiel 1 erhält man durch Umsetzung von 4-p-Chlorbiphenyl-isopropyl-keton mit Methyljodid in 70%iger Ausbeute 4-p-Chlorbiphenylyl-tert.-butyl-keton vom Schmelzpunkt 99° C.

Antimikrobielle Austestungen:

Beispiel A
Antimykotische in-vitro-Wirksamkeit

Versuchsbeschreibung

Die in-vitro-Prüfungen wurden im Reihenverdünnungstest mit Keiminokula von durchschnittlich $5 \times 10$ Keimen/ml Substrat durchgeführt. Als Nährmedizin dienten

a) für Dermatophyten und Schimmelpilze:
Sabourand's milieu d'épresive
b) für Hefen:
Isotonic Sensitest-Broth von Oxid.

Die Bebrütungstemperatur betrug 28° C; Bebrütungszeit war 24 Stunden bei Hefen und 96 Stunden bei Dermatophyten und Schimmelpilzen.
In diesem Test zeigten die erfindungsgemäßen Verbindungen bei zahlreichen Dermatophyten, Schimmelpilzen und Hefen gute MHK-Werte.

Beispiel B
Antimikrobielle in-vivo-Wirksamkeit (oral) bei Mäuse-Candidose

Versuchsbeschreibung

Mäuse vom Typ SPF-$CF_1$ wurden intravenös mit $1-2 \times 10^6$ logarithmisch wachsenden Candida-Zellen, die in physiologischer Kochsalzlösung suspendiert waren, infiziert. Eine Stunde vor und sieben Stunden nach der Infektion werden die Tiere mit jeweils 50–100 mg/kg Körpergewicht der Präparate oral behandelt.

Ergebnis:

Unbehandelte Tiere starben 3 bis 6 Tage post infektionem. Die Überlebensrate am 6. Tag post infektionem betrug bei unbehandelten Kontrolltieren etwa 5%.
In diesem Test zeigte die erfindungsgemäße Verbindung 2 eine sehr gute Wirkung, während das bekannte Miconazol® keine Wirkung zeigte.

**Patentansprüche:**

1. Mittel zur Behandlung von Mykosen, gekennzeichnet durch einen Gehalt an mindestens einem Hydroxybutyl-imidazolderivat der Formel

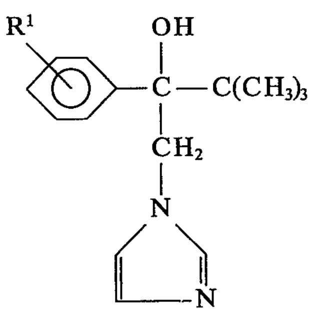

(I)

in welcher

$R^1$ für gegebenenfalls durch Halogen, Alkyl mit 1–2 Kohlenstoffatomen oder Alkoxy mit 1–2 Kohlenstoffatomen substituiertes Phenyl steht und/oder deren physiologisch verträglichen Säureadditionssalzen.

2. Mittel zur Behandlung von Mykosen, gekennzeichnet durch einen Gehalt an 1-(4-Biphenyl)-1-(tert.-butyl)-2-(imidazol-1-yl)-ethan-1-ol.

3. Mittel zur Behandlung von Mykosen, gekennzeichnet durch einen Gehalt an 1-(4-p-Chlorbiphenylyl)-1-(tert.-buyl)-2-(imidazol-1-ol)-ethanol-1-ol.

4. Verfahren zur Herstellung eines Mittels zur Behandlung von Mykosen, dadurch gekennzeichnet, daß man Hydroxybutyl-imidazole gemäß der Formel in Anspruch 1 mit inerten, nichttoxischen, pharmazeutisch geeigneten Trägerstoffen vermischt.

## Claims

1. Antimycotic agent, characterised in that it contains at least one hydroxybutyl-imidazole derivative of the formula

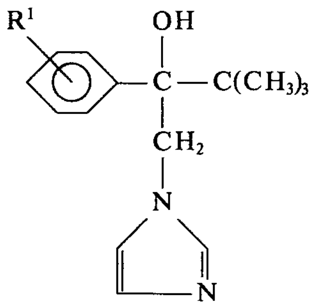

(I)

in which

$R^1$ represents phenyl which is optionally substituted by halogen, alkyl having 1–2 carbon atoms or alkoxy having 1–2 carbon atoms,

and/or their physiologically acceptable acid addition salts.

2. Antimycotic agent, characterised in that it contains 1-(4-biphenylyl)-1-(tert.-butyl)-2-(imidazol-1-yl)-ethan-1-ol.

3. Antimycotic agent, characterised in that it contanis 1-(4-p-chlorobiphenylyl)-1-(tert.-butyl)-2-imidazol-1-ol)-ethanol-1-ol.

4. Process for the preparation of an antimycotic agent, characterised in that hydroxybutyl-imidazoles according to the formula in Claim 1 are mixed with inert, non-toxic, pharmaceutically suitable excipients.

## Revendications

1. Agents pour le traitement de mycoses, caractérisés en ce qu'ils contiennent au moins un dérivé d('hydroxybutylimidazole de formule

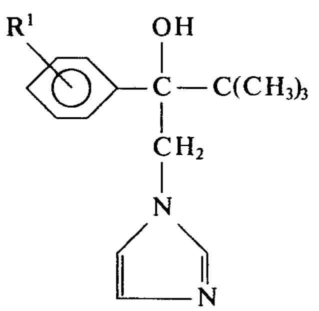

(I)

dans laquelle

$R^1$ représente un reste phényle éventuellement substitué par un halogène ou un alkyle en $C_1-C_2$ ou alcoxy en $C_1-C_2$ et/ou leurs sels d'addition d'acides physiologiquement compatibles.

2. Agents pour le traitement de mycoses, caractérisés en ce qu'ils contiennent du 1-(4-biphénylyl)-1-(tert-butyl)-2-(imidazole-1-yl)-éthane-1-ol.

3. Agents pour le traitement de mycoses, caractérisés en ce qu'ils contiennent du 1-(4-chlorobiphénylyl)-1-(tert-butyl)-2-(imidazole-1-ol)-éthanol-1-ol.

4. Procédé pour la préparation d'un agent pour le traitement de mycoses, caractérisé en ce que l'on mélange des hydroxybutylimidazoles selon la formule de la revendication 1 avec des supports inertes non toxiques appropriés en pharmacie.